(19) Europäisches Patentamt / European Patent Office / Office européen des brevets

(11) EP 3 264 347 A1

(12) EUROPEAN PATENT APPLICATION
published in accordance with Art. 153(4) EPC

(43) Date of publication:
03.01.2018 Bulletin 2018/01

(51) Int Cl.:
G06Q 10/10 $^{(2012.01)}$     G06Q 50/22 $^{(2018.01)}$
G06Q 50/24 $^{(0000.00)}$     A61B 5/00 $^{(2006.01)}$

(21) Application number: 15883347.5

(22) Date of filing: 24.11.2015

(86) International application number:
PCT/JP2015/082915

(87) International publication number:
WO 2016/136057 (01.09.2016 Gazette 2016/35)

(84) Designated Contracting States:
AL AT BE BG CH CY CZ DE DK EE ES FI FR GB
GR HR HU IE IS IT LI LT LU LV MC MK MT NL NO
PL PT RO RS SE SI SK SM TR
Designated Extension States:
BA ME
Designated Validation States:
MA MD

(30) Priority: 25.02.2015 JP 2015035389

(71) Applicant: Fujitsu Limited
Kawasaki-shi, Kanagawa 211-8588 (JP)

(72) Inventors:
• HOTTA, Shinji
Kawasaki-shi
Kanagawa 211-8588 (JP)
• INOMATA, Akihiro
Hayes, Middlesex UB4 8FE (JP)

(74) Representative: Haseltine Lake LLP
Lincoln House, 5th Floor
300 High Holborn
London WC1V 7JH (GB)

(54) **CORRELATION DETERMINATION PROGRAM, CORRELATION DETERMINATION METHOD, AND CORRELATION DETERMINATION DEVICE**

(57) A correlation determination program for causing a computer to execute a process including calculating a time interval between an occurrence time of one of first type events and an occurrence time of each of second type events based on information including the occurrence times of the first type events and the second type events, for each of the first type events; comparing, for each of ranges of values relating to the time interval, a first rate of the number of time intervals within the range among a set of time intervals to a size of the range and a second rate of the number of time intervals outside the range among the set to a size of a range obtained by excluding the range from the predetermined time; and determining presence or absence of a correlation between the first type and second type events.

FIG.9

**Description**

TECHNICAL FIELD

A certain aspect of the embodiments discussed herein relates to a correlation determination program, a correlation determination method, and a correlation determination apparatus

BACKGROUND ART

In medical practice, treatments are generally conducted based on observational information in hospitals.

With the advent of wearable sensors and the like, computers are now enabled to collect events (hereinafter referred to as "events") indicated by sensor data (accelerations, speeds, heart rates, blood pressures, etc.) relating to activities during daily life for each individual. Further, individuals or physicians, etc. may input, into a computer, events (drug administration, dietary intake, etc.) relating to activities during daily life.

As described above, observation of collected or input events with respect to activities of patients during daily life may enable physicians to clarify conditions or circumstances that are likely to cause functional abnormality in the patients, leading to potentially effective medical treatments. For example, a physician may be able to clarify that abnormality in a patient's blood pressure takes place after the patient takes a certain drug, and the physician may discontinue the drug as the effective treatment for this condition.

In general, it is assumed that an event to be focused on is specified for each function abnormality (hereinafter referred to as "focused event") based on typical medical knowledge. For example, in a case of a patient with abnormality in the heart, one needs focus on the abnormality of the electrocardiogram waveform, or in a case of finding risks of falling, one needs to focus such as on a decrease in walking speed or on a staggering gait occurrence.

Focusing on such an event may enable extraction of some background of a cause of the focused event, that is, another event correlated with the focused event (hereinafter referred to as a "related event"), which may provide effective knowledge for remediating the functional abnormality.

With respect to events related to computer systems, attempts have been made to conduct a study of extracting, as a related event, an event for which the occurrence frequency immediately before the focused event is equal to or higher than a threshold.

RELATED ART DOCUMENTS

PATENT DOCUMENTS

Patent Document 1: Japanese Laid-open Patent Publication No. 2008-41041
Patent Document 2: Japanese Laid-open Patent Publication No. 2004-157614
Patent Document 3: Japanese Laid-open Patent Publication No. 2007-48200
Patent Document 4: Japanese Laid-open Patent Publication No. 2013-131170
Patent Document 5: Japanese Patent Application No. 2001-540710
Patent Document 6: Japanese Laid-open Patent Publication No. 2011-209908

DISCLOSURE OF INVENTION

PROBLEM TO BE SOLVED BY INVENTION

However, with respect to activities during the daily life of a person, in particular, a timing of occurrence of a related event is not necessarily immediately before the focused event. For example, blood pressure may decrease between 5 and 60 minutes after meal. Further, it may take between 5 and 15 minutes to return to a normal heart rate after exercise. In addition, heart rate may rise in 30 minutes to 90 minutes after a meal, due to energy consumption by digestion.

As described above, there is a high possibility of failing to extract related events having a long occurrence interval with respect to the focused event only by observing a period immediately before the focused event.

In view of this, in order to enable extraction of related events having long occurrence intervals, consideration may be made to extract, as a related event, an event whose occurrence frequency in a long term range from the occurrence of the focused event is equal to or higher than a threshold. However, in this case, events related to actions normally performed as daily activities may be extracted undesirably. For example, with respect to a person having a lifestyle habit of going to bed within 5 hours after having dinner, there is a possibility of extracting dinner intake as a related event in a case of going to bed being determined as a focused event.

In addition, in a case where a related event is extracted depending only on the occurrence frequency of the related event, there is a high possibility of extracting constantly occurring events, such as "breathing" and "blinking", as related events of a certain event.

MEANS FOR SOLVING PROBLEMS

Accordingly, it is one aspect of an object to improve extraction accuracy of an event having a correlation with a certain event.

According to an aspect of the embodiments,
a correlation determination program for causing a computer to execute a process is provided. The process includes calculating a time interval between an occurrence time of one of first type events and an occurrence time of each of second type events based on information including the occurrence times of the first type events and the second type events, on a first type event basis, each of the second type events occurring within a predetermined time from a corresponding one of the occurrence times of the first type events; comparing, for each of a plurality of ranges of values relating to the time interval, the plurality of ranges differing from one another in at least one of a minimum value and a maximum value, a first rate of the number of time intervals within a corresponding one of ranges among a set of time intervals with respect to a size of the range and a second rate of the number of time intervals outside the corresponding range among the set of time intervals with respect to a size of a range obtained by excluding the range from the predetermined time; and determining presence or absence of a correlation between the first type event and the second type event.

EFFECT OF INVENTION

According to one aspect of the disclosure, the extraction accuracy of an event having a correlation with a certain event may be improved.

BRIEF DESCRIPTION OF DRAWINGS

FIG. 1 includes diagrams illustrating a method of reading drawings used in present embodiments;
FIG. 2 is a diagram illustrating a first idea acting as a basis in an embodiment of the present invention;
FIG. 3 is a diagram illustrating a second idea acting as a basis in an embodiment of the present invention;
FIG. 4 is a diagram illustrating a third idea acting as a basis in an embodiment of the present invention;
FIG. 5 includes first diagrams illustrating an outline of a related event determination method in an embodiment of the present invention;
FIG. 6 is a second diagram illustrating an outline of the related event determination method according to the embodiment of the present invention;
FIG. 7 is a diagram illustrating a hardware configuration example of a correlation determination apparatus according to an embodiment of the present invention;
FIG. 8 is a diagram illustrating a functional configuration example of the correlation determination apparatus according to the embodiment of the present invention;
FIG. 9 is a flowchart illustrating an example of a process executed by a correlation determination apparatus;
FIG. 10 includes diagrams illustrating examples of respective time series data of a focused event and a candidate event;
FIG. 11 is a diagram illustrating a configuration example of a parameter storage;
FIG. 12 is a diagram illustrating an example of a calculation result of a time interval between a focused event and a candidate event;
FIG. 13 is a diagram illustrating generation of a determination window;
FIG. 14 is a diagram illustrating an example of data specified by a generation process of a determination window;
FIG. 15 is a flowchart illustrating an example of a process of determining whether there is a correlation between a focused event and a candidate event with respect to one determination window;
FIG. 16 is a diagram illustrating a calculation example of $Z1_{in}$ and $Z1_{out}$ and a ratio R1 for each case;
FIG. 17 is a diagram illustrating an example of a method of obtaining a threshold T_TH according to a start point of a determination window;
FIG. 18 is a diagram illustrating an example of a method of calculating a ratio R2; and

FIG. 19 is a diagram illustrating a configuration example of a related event storage.

EMBODIMENTS FOR IMPLEMENTING THE INVENTION

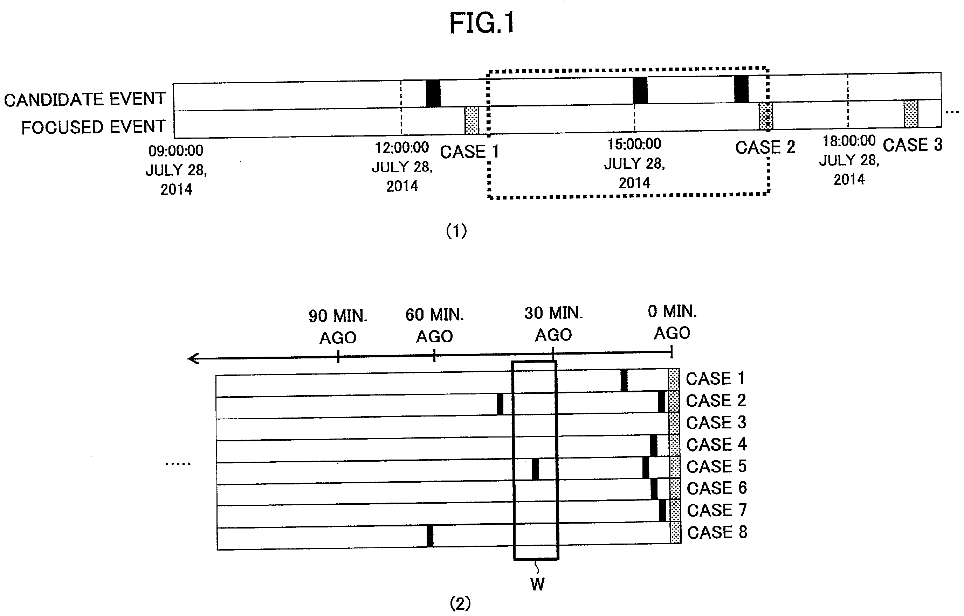

Embodiments of the present invention will be described below with reference to the accompanying drawings. First, the way of reading the drawings used in the present embodiments will be described. FIG. 1 includes diagrams illustrating a method of reading drawings used in the present embodiment.

In the present embodiments, it is assumed that data relating to activities in daily life of a person are detected by a sensor attached to the person or carried by the person, or input by the person, and such data are collected by a computer. In such a condition, a method of extracting an event having a correlation or relationship with a certain event (hereinafter unified as the term "correlation") will be described. Note that a "certain event" hereinafter is referred to as a "focused event", and an event correlated with the focused event is referred to as a "related event".

To extract a related event, an event subject to being determined as a focused event or a related event (hereinafter referred to as "candidate event") is initially specified.

(1) of FIG. 1 illustrates an example in which an occurrence point of a candidate event and an occurrence point of a focused event, both of which are specified based on collected data, are arranged on a timeline. That is, a horizontal direction of (1) of FIG. 1 indicates a time axis, and time progresses from left to right. In each row, a point at which a filled rectangle is arranged indicates the occurrence point of the candidate event or the focused event. In the present embodiment, each occurrence of a focused event is referred to as a "case n". Further, "n" indicates the occurrence order of the focused events in a period where the focused events are subject to observation. In (1) of FIG. 1, cases 1 to 3 are illustrated, and cases subsequent as of a case 4 are omitted.

In (2) of FIG. 1, for each case n, a candidate event that has occurred within a predetermined period from the occurrence point of the case n is plotted on the basis of the occurrence point of the case n. That is, in (2) of FIG. 1, each case is arranged in a vertical direction. In a row for each case n, the occurrence point of the corresponding candidate event, which has occurred within a predetermined period from the occurrence point of a focused event relating to the case n, is plotted. For example, as apparent from a range indicated by a broken-line rectangle in (1) of FIG. 1, the candidate event occurs twice in a predetermined period before the occurrence of the case 2. Accordingly, in (2) of FIG. 1, the occurrence points of these two candidate events are plotted, respectively, in the row corresponding to case 2.

Further, in (2) of FIG. 1, a solid-line rectangle indicated by a symbol W is a window (hereinafter referred to as "determination window") indicating a duration in which a candidate event is subject to observation. In the present embodiment, while shifting the determination window W in a horizontal direction, the occurrence frequency (hereinafter referred to as unit "frequency") of candidate events in the determination window W is measured. The formula for calculating the frequency of certain events is, for example, as follows.

Frequency = (the number of cases in which an event occurred one or more times within the determination window W) ÷ the number of all cases.

For example, the frequency of candidate events in (2) of FIG. 1 is 1 ÷ 8 = 0.125. Note that, in the determination window W, even if the candidate event were to occur twice with respect to Case 5, the frequency of the candidate event would still be 0.125.

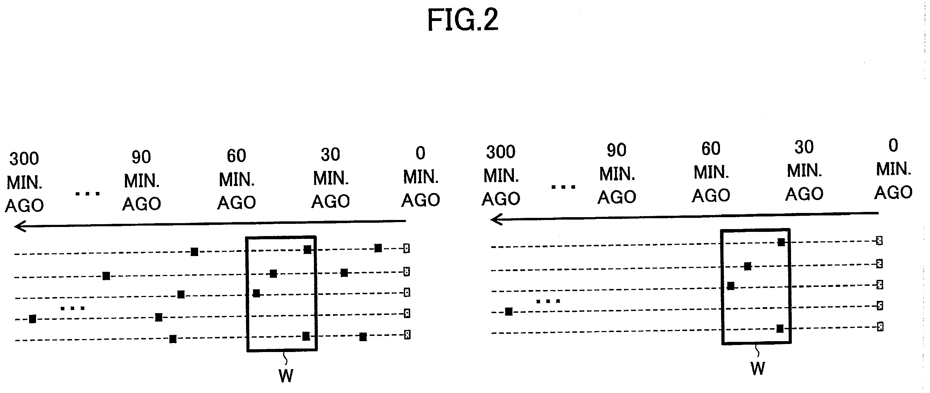

Next, the basic idea in the present embodiment will be described. FIG. 2 is a diagram illustrating a first idea acting as a basis in the embodiment of the present invention.

The first idea determines that the correlation between the candidate event and the focused event is high in a case of the frequency in the determination window W being much larger than the frequency outside the determination window W, even if the frequency in the determination window W is small. That is, in FIG. 2, the frequency in the determination window W is the same in a left side condition and a right side condition, but the ratio of the frequency in the determination window W to the frequency outside the determination window W is higher in the right side condition. Accordingly, in FIG. 2, a correlation between the candidate event and the focused event is considered to be higher in the right side condition than the left side condition.

For example, it is assumed that the focused event is the start of sleeping and the candidate event is the end of a meal. In the left side condition, the frequency of starting to sleep between 30 to 45 minutes from after meals (a time period of the determination window W) is 4 ÷ 5 = 0.8, and a similar frequency is observed in other time periods. However, in the right side condition, although the frequency of starting to sleep between 30 and 45 minutes (a time period of the determination window W) from after meals is also 4 ÷ 5 = 0.8, a similar frequency is not observed in other time periods. In such a case, it is considered that the correlation between the end of a meal and the start of sleep is higher in the right side condition.

However, according to the first idea, as the duration of the determination window W is wider, there may be a high possibility that the frequency within the determination window W will increase. Hence, in order to supplement the first idea, a second idea is introduced in this embodiment.

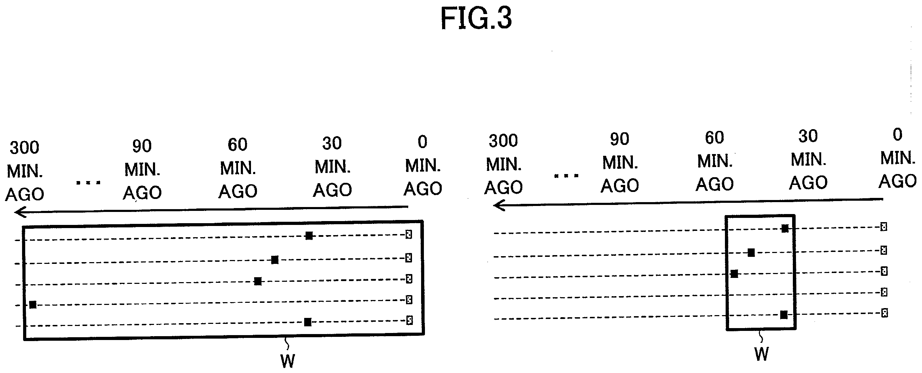

FIG. 3 is a diagram illustrating the second idea acting as a basis in the embodiment of the present invention. The second idea indicates a case where the difference is small between the frequency of the determination window W having a relatively narrow duration and the frequency of the determination window W having a relatively wide duration, and determines that the former has higher correlation even if the frequency of the former is smaller.

For example, in FIG. 3, the frequency in the determination window W in the left side condition is higher than the frequency in the determination window W in the right side condition; however, a width of the determination window W in the right side condition is much narrower than a width of the determination window W in the left side condition. Hence, in this case, the correlation is considered to be higher in the determination window W in the right side condition. The determination window W in the left side condition illustrates that the frequency of starting to sleep between 0 to 300 minutes after meals is high. The determination window W in the left side condition illustrates that the frequency of starting to sleep between 30 to 45 minutes after meals is high. In such a case, it is considered that the correlation between the end of a meal and the start of sleep is higher in the right side condition.

However, as an interval increases between a time at which a related event occurs and a time at which the focused event occurs, variability in the occurrence time of the related event with respect to the focused event in each case will increase. For example, when a case where side effect X develops within 5 minutes after taking medicine A is compared with a case where side effect Y develops 3 hours after taking medicine B, the latter case has high possibility of having variability in timing of developing the side effect. This is because physiological characteristics of a person, such as a physical condition, may vary with the day when the medicine is taken. Further, the longer the interval between the related event and the focused event, the higher the possibility of adding another action that increases the interval between the related event and the focused event. For example, in comparing people who go to bed within 30 minutes after taking medicine, and people who go to bed after 3 hours after taking medicine, the latter people may have high possibility of varying time going to bed due to activities performed during 3 hours.

With the first and second ideas alone, for example, even if the correlation between the above "taking medicine A" and "development of side effect X" and the correlation between taking medication B" and " development of side effect Y" is the same degree as such, there may be a possibility that the former is determined to have a higher correlation. Hence, in order to supplement the second idea, a third idea is introduced in this embodiment.

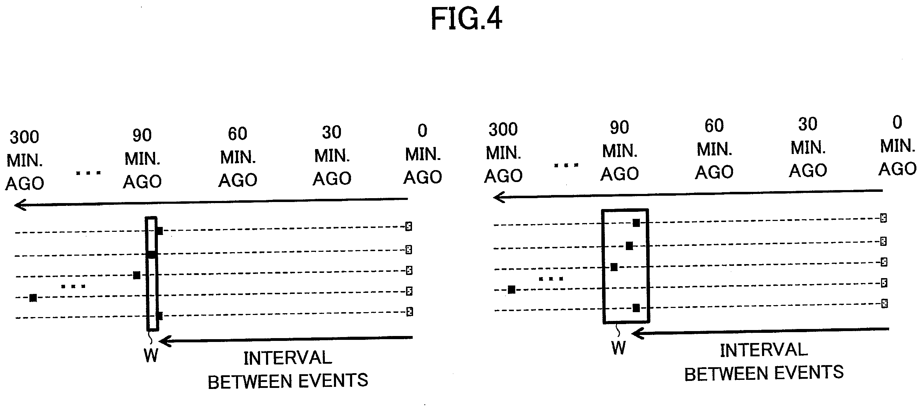

FIG. 4 is a diagram illustrating the third idea acting as a basis in the embodiment of the present invention. The third idea estimates a possibility of having a correlation between the candidate event and the focused event when the duration of the determination window W is large and the interval between the candidate event and the focused event is substantially long.

In a case of the left side condition of FIG. 4, the frequency of candidate events in the determination window W is 1/5 = 0.2. However, in FIG. 4, since the candidate event group is about 90 minutes separate from the focused event group, as illustrated on the right side of FIG. 4, the candidate event group is allowed to vary and the duration of the determination window W is increased; as a result, the frequency of candidate events in the determination window W is $4 \div 5 = 0.8$.

In the present embodiment, it is determined whether the candidate event is a related event based on the above-described first to third ideas. Next, the points of determining the related event in the present embodiment will be described.

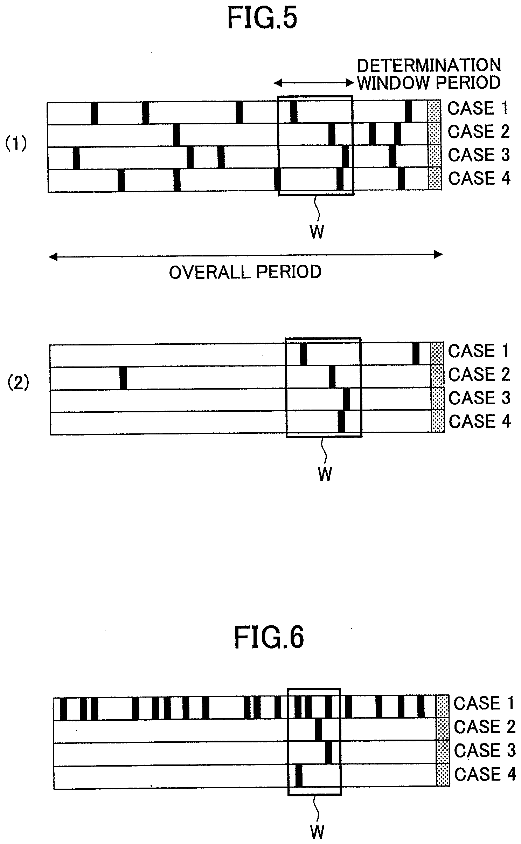

FIG. 5 includes first diagrams illustrating an outline of a related event determination method in an embodiment of the present invention.

In this embodiment, two or more types of determination windows W mutually differing in at least one of the duration and the position are used. For each determination window W, the occurrence rate of the candidate events within the determination window W and the occurrence rate of the candidate events outside the determination window W are calculated. In any of the determination windows W, when the ratio of the former occurrence rate (candidate event within the determination window W) to the latter occurrence rate (candidate event outside the determination window W) is equal to or greater than the threshold R_TH, the candidate event is determined to be a related event. Note that the occurrence rate of candidate events within the determination window W is calculated by dividing the number of occurrences of candidate events in the determination window W by the duration of the determination window W. Further, the occurrence rate of the candidate events outside the determination window W is calculated by dividing the number of occurrences of the candidate event outside the determination window W by a period obtained by subtracting the duration of the determination window W from the overall period (a period in a horizontal direction in FIG. 5. Accordingly, with respect to FIG. 5, there is a higher possibility of determining in (2) of FIG. 5 that the candidate event is a related event than in (1) of FIG. 5. Note that the process described in FIG. 5 is referred to as "point 1".

However, depending on the occurrence condition of the event, there are cases in which point 1 may not be valid. To supplement such a case in the present embodiment, a process described in FIG. 6 is executed.

FIG. 6 is a second diagram illustrating an outline of the related event determination method according to the embodiment of the present invention. In FIG. 6, a candidate event is assumed to have occurred 100 times in a case 1. In contrast, the candidate event is assumed to have occurred only once in each of cases 2 to 4. In such a case, there is a high possibility of calculating the ratio by point 1 to be less than the threshold R_TH. Accordingly, there is a high possibility of determining the candidate event being not a related event. However, in observing cases 2 to 4, the possibility of the candidate event being a related event is considered to be very high.

Hence, in the present embodiment, before the execution of point 1, the occurrence rate of the candidate events within the determination window W and the occurrence rate of the candidate events outside the determination window W are calculated for each case. As a result, when a ratio of the cases, for which the ratio of the former occurrence rate to the latter occurrence rate is equal to or greater than a threshold $\alpha$, is equal to or greater than a threshold $\beta$, point 1 is not executed and the candidate event is determined to be a related event.

The process described in FIG. 6 is referred to as "point 2". Point 1 and Point 2 are processes based on the first idea.

In the present embodiment, two or more types of determination windows W mutually differing in at least one of a duration and a position are used; however point 1 is not valid with respect to the determination windows W of all the durations. That is, as described with reference to FIG. 3, even when the ratio by point 1 is the same with respect to the determination window W having a short duration and the determination window W having a long duration, the ratio with respect to the determination window W having a short duration needs to be evaluated as having a high correlation.

Hence, in the present embodiment, in point 2, when the candidate event is not determined to be a related event and the duration of the determination window W is equal to or less than the threshold T_TH, a process called point 3 is executed to enable the process of point 1 to be valid. Note that point 3 is a process corresponding to the third idea.

Furthermore, as described with reference to FIG. 4, the longer the interval between the focused event and the related event, the greater the allowable range should be with respect to the deviation between the candidate events. Hence, in the present embodiment, the threshold T_TH used in point 2 is changed according to the position of the determination window W. More specifically, as the position of the determination window W becomes farther from the focused event, the threshold HT2 becomes larger. Hereinafter, the process related to the change of the threshold T_TH is referred to as point 4.

The following illustrates a correlation determination apparatus 10 configured to determine related events based on the points 1 to 4.

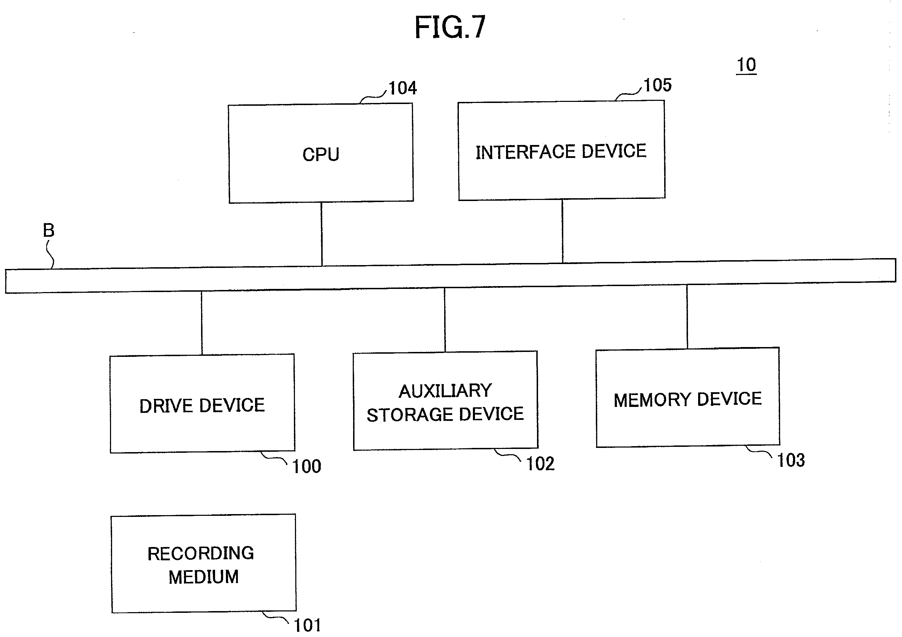

FIG. 7 is a diagram illustrating an example of a hardware configuration of a correlation determination apparatus according to an embodiment of the present invention. The correlation determination apparatus 10 in FIG. 7 includes a drive device 100, an auxiliary storage device 102, a memory device 103, a CPU 104, an interface device 105, and the like.

A program for implementing a process in the correlation determination apparatus 10 is provided from a recording medium 101. When the recording medium 101 storing the program is set in the drive device 100, the program is installed from the recording medium 101 to the auxiliary storage device 102 via the drive device 100. Note that it is not always necessary to install the program from the recording medium 101; however, the program may be downloaded from another computer via the network. The auxiliary storage device 102 stores the installed programs and stores necessary files, data, and the like.

The memory device 103 reads a program from the auxiliary storage device 102 and stores the read program when receiving an instruction to activate the program. The CPU 104 implements a function of the correlation determination apparatus 10 according to the program stored in the memory device 103. The interface device 105 is used as an interface for connecting to the network.

Note that an example of the recording medium 101 is a removable recording medium such as a CD-ROM, a DVD disk, or a USB memory. Further, examples of the auxiliary storage device 102 include an HDD (Hard Disk Drive), a flash memory, and the like. Each of the recording medium 101 and the auxiliary storage device 102 corresponds to a computer-readable recording medium.

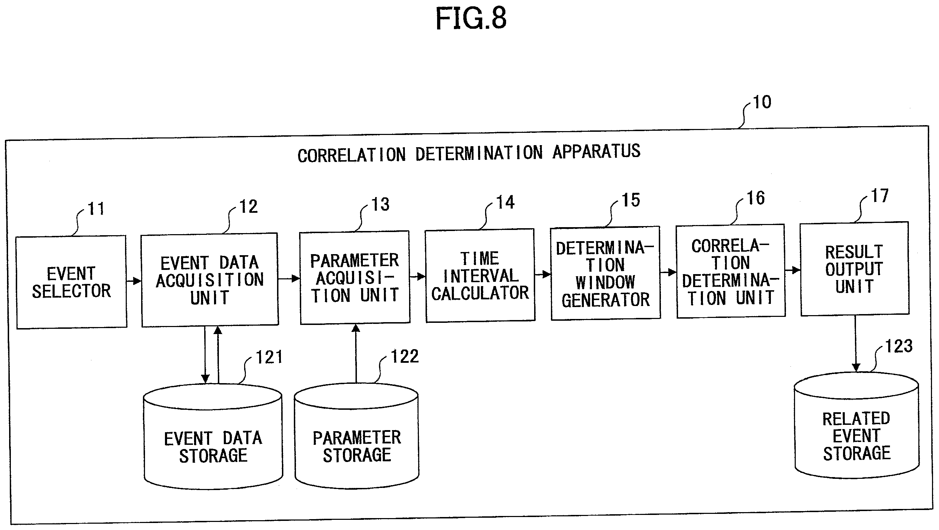

FIG. 8 is a diagram illustrating a functional configuration example of the correlation determination apparatus according to the embodiment of the present invention. In FIG. 8, the correlation determination apparatus 10 includes an event selector 11, an event data acquisition unit 12, a parameter acquisition unit 13, a time interval calculator 14, a determination window generator 15, a correlation determination unit 16, a result output unit 17, and the like. These units are implemented by a process to be executed by the CPU 104, which is caused by one or more programs installed in the correlation determining apparatus 10. The correlation determination apparatus 10 also uses an event data storage 121, a parameter storage 122, a related event storage 123, and the like. Each of these storages may be implemented by using, for example, the auxiliary storage device 102, or a storage device or the like connectable to the correlation determination apparatus 10 via a network.

The event selector 11 selects a type of an event to be the focused event and a type of an event to be the candidate event.

The event data acquisition unit 12 acquires event data on the focused event and event data on the candidate event from the event data storage 121. The event data storage 121 stores event data on each of the events in time series. Each of event data includes, for example, the type of the event, the occurrence date and time of the event, and the like.

The parameter acquisition unit 13 acquires from the parameter storage 122 parameters related to the determination window W used for the process of determining the presence or absence of a correlation between the focused event and the candidate event. Based on the event data group acquired by the event data acquisition unit 12, the time interval calculator 14 calculates a time interval between the occurrence date and time of the focused event and the occurrence date and time of each candidate event that has occurred within a predetermined time from the occurrence of the focused event.

The determination window generator 15 generates two or more determination windows W mutually differing in at least one of a duration and a position. The correlation determination unit 16 determines the presence or absence of a correlation between the focused event and the candidate event on the basis of the above points 1 to 4 by using the two or more determination windows W. The result output unit 17 outputs the determination result obtained by the correlation determination unit 16. For example, the result output unit 17 stores in the related event storage 123 information on a candidate event (i.e., related event) determined to have a correlation with respect to the focused event.

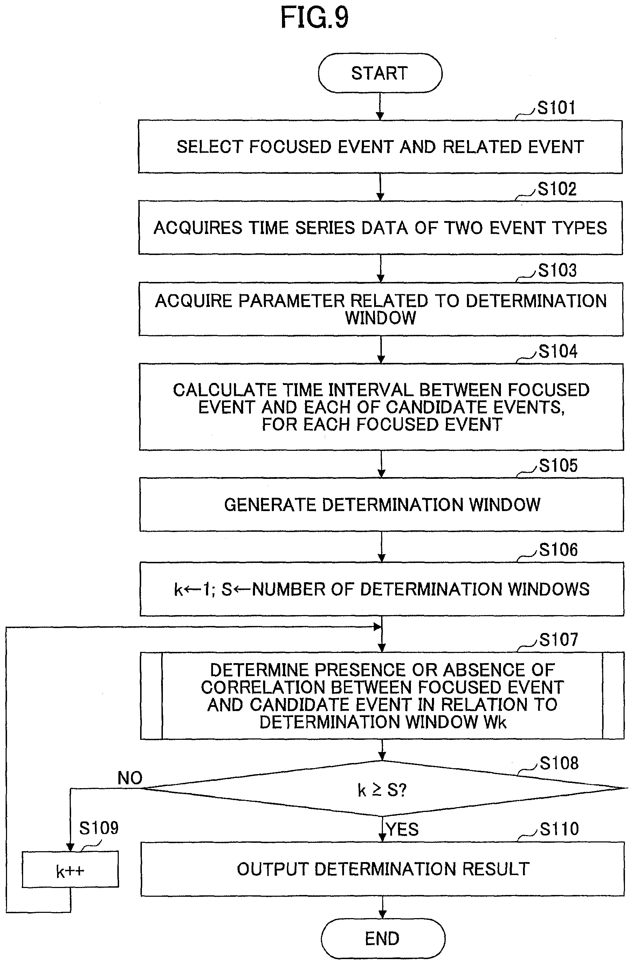

The following describes a process executed by the correlation determination apparatus 10. FIG. 9 is a flowchart illustrating an example of a process executed by the correlation determination apparatus.

In step S101, the event selector 11 selects one type to be a focused event and one or more types to be candidate events from among two or more event types. The type of the focused event and the types of the candidate events may be input by a user. For example, it is assumed that a physician prescribing a certain medicine X for a patient A is a user. The user desires to know how administration of medicine X affects the patient A. In this case, for example, "take medicine X" is defined as a focused event, and "dizziness", "heart rate rises to 20 bpm or more", "body temperature rises to 38 degrees or more", "systolic blood pressure rises to 140 mmHg or more" may be selected as candidate events. Step S103 and subsequent steps are executed for all candidate events; however, one candidate event "dizziness" will be described as an example in the present embodiment, for the sake of convenience. In the following, simply referring to a "candidate event" indicates one candidate event to be processed among the selected candidate events.

Subsequently, the event data acquisition unit 12 acquires event data on the patient A, which is the event data on the focused event and the event data on the candidate event from the event data group stored in the event data storage 121. The event data acquisition unit 12 generates respective time series data based on the event data extracted for each of the focused event and the candidate event (S102). In the present embodiment, the data collected by the sensor attached to the patient A, the data input by the patient A or the physician (i.e., the user), etc. are stored in time series as event data on the patient A, and are stored in the event data storage 121.

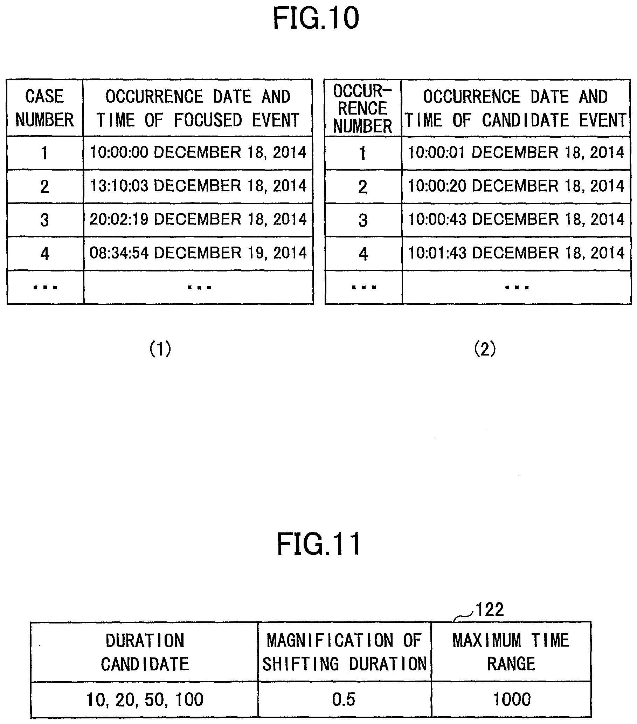

FIG. 10 includes diagrams illustrating examples of respective time series data of focused events and a candidate event. (1) of FIG. 10 illustrates time series data of focused events. The time series data includes a case number and occurrence date and time for each focused event that has occurred. The case number is a number indicating the occurrence order of the focused event, and is assigned by the event data acquisition unit 12, for example.

In addition, (2) of FIG. 10 illustrates time series data of candidate events. The time series data includes a case number and occurrence date and time for each candidate event that has occurred. The occurrence number is a number indicating the occurrence order of the candidate events, and is assigned by the event data acquisition unit 12, for example. The respective time series data of the focused events and the candidate events are data indicating information similar to the information illustrated in (1) of FIG. 10.

Note that the case number and the occurrence number do not indicate the correspondence relationship between the focused event and the candidate event. Each of the case number and the occurrence number indicates the order of occurrence within a corresponding one of event groups.

Subsequently, the parameter acquisition unit 13 acquires parameters related to the determination window W from the parameter storage 122 (S103).

FIG. 11 is a diagram illustrating a configuration example of a parameter storage. As illustrated in FIG. 11, the parameter storage 122 stores a candidate duration, a magnification of a shifting duration, a maximum time range, and the like.

The candidate duration is a candidate duration of the determination window W. The magnification of the shifting duration is a parameter for determining a shift duration of the determination window W. A value obtained by multiplying the duration of the determination window W by the magnification of the shifting duration is a shifting duration of the determination window W. Note that the shifting duration of the determination window W indicates an interval between the position of the determination window W before shifting the position of the determination window W and the position of the determination window W after shifting the position of the determination window W. The maximum time range is a parameter indicating an extraction range of the candidate event with respect to the focused event relating to each of the cases. For example, the maximum time range that may be considered to be affected by the focused event is specified. In the present embodiment, the unit of duration and of the maximum time range is seconds.

The candidate duration and the maximum time range may be set by utilizing prior knowledge in a case where there is a certain extent of prior knowledge such as empirical knowledge of the user (physician) (knowledge such as the effect of medicine X appearing within 1 to 2 hours after taking the medicine X). When the effect of medicine X appears within 1 to 2 hours after taking the medicine X, for example, a value indicating 2 hours may be set as the maximum time range.

Subsequently, the time interval calculator 14 calculates, for each of the focused events, a time interval (the absolute value of the difference in the occurrence date and time) from the occurrence date and time of the corresponding focused event to each of the candidate events occurring within the maximum time range (S104). In the present embodiment, since the focused event (taking medicine X) is an event that occurs earlier than the candidate event (dizziness), "within the maximum time range from the occurrence date and time of the focused event" indicates a duration after the occurrence date and time of the focused event. In a case where the focused event indicates an event occurring after the candidate event, the maximum time range from the occurrence date and time of the focused event indicates a duration before the occurrence date and time of the focused event. Further, although the calculated time interval does not necessarily need to be an absolute value, in the present embodiment, an absolute value is used for the time interval in this embodiment in order to simplify the illustration (i.e., for convenience of illustration) by excluding a case where the time interval is negative.

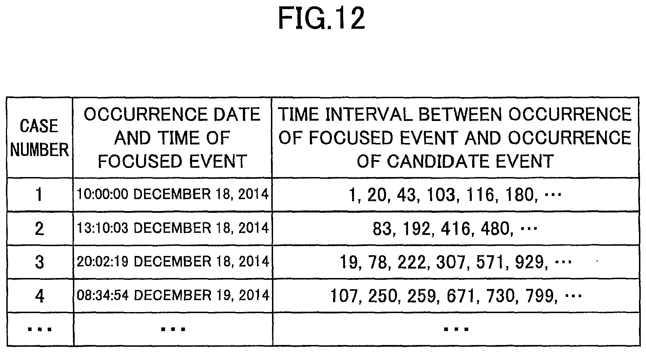

FIG. 12 is a diagram illustrating an example of a calculation result of a time interval between a focused event and a candidate event. FIG. 12 illustrates the calculation result of the time interval between the focused event and each of the candidate events occurring within the maximum time range from the occurrence date and time of the focused event, for each of the focused events illustrated in (1) of FIG. 10. In the present embodiment, the unit of time interval is seconds. Note that the information illustrated in FIG. 12 corresponds to the information illustrated in (2) of FIG. 1.

In general, the maximum time range is much shorter than an occurrence interval of each of the focused events. For example, when the timing of taking medicine X is before bedtime (i.e., once a day) and the effect of medicine X appears within 3 hours, the time interval is calculated with respect to the candidate event in the three hours in step S104. Accordingly, it is unlikely that duplicated time intervals between the two or more focused events and the same candidate event are calculated. Note that when such duplication may occur, the time interval may be calculated by removing the duplication.

Subsequently, the determination window generator 15 generates a determination window W based on the parameter relating to the determination window W (FIG. 11) (S105). Specifically, for example, 0 is set as an initial value of a start point (the minimum value of the range of the determination window W) for each of the determination windows W having the four durations of 10, 20, 50, and 100, and the determination window W is generated as the start point is shifted by the shifting duration within the maximum time range. The end point of the determination window W (the maximum value of the range of the determination window W) is obtained by adding the duration of the determination window W (the size of the determination window W) to the start point of the determination window W. Further, the shifting duration is specified by multiplying the duration of the determination window W by the magnification of the shifting duration. As a result, two or more determination windows W differing from one another in at least one of the start point (minimum value) and the end point (maximum value) are generated. Note that the initial value at the start point is not necessarily 0. For example, insofar as the initial value is equal to or greater than 0 and equal to or less than the minimum value of the time interval calculated in step S104, the origin at the start point may be any value.

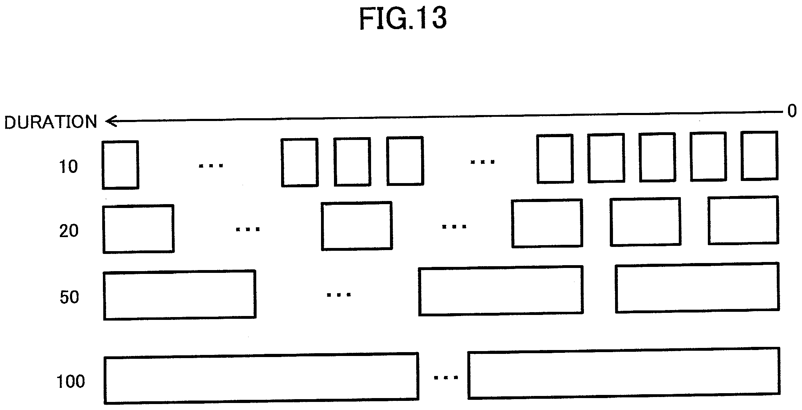

FIG. 13 is a diagram illustrating generation of a determination window. In FIG. 13, determination windows W having varying candidate event durations with respect to the start point are enumerated. Note that the determination windows W enumerated in FIG. 13 do not strictly represent the determination windows W generated based on the parameters illustrated in FIG. 11. In the present embodiment, since the magnification of the shifting duration is 0.5 (i.e., less than 1), the determination windows W shifted by one duration from each other have a mutually overlapping range.

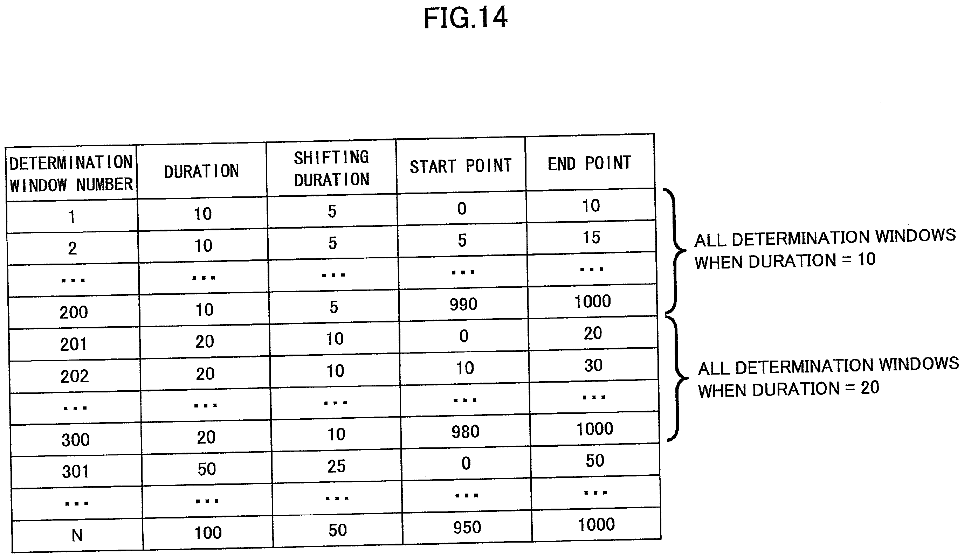

Note that in step S105, for each of the generated determination windows W, the start point and the end point and the like of the determination window W are specified. FIG. 14 is a diagram illustrating an example of data specified by a generation process of a determination window. As illustrated in FIG. 14, in step S105, a determination window number, duration, a shifting duration, a start point, an end point, and the like are specified for each determination window W.

The determination window number is identification information of each determination window W. The duration and the shifting duration are duration and shifting duration used for generating the determination window W. The start point is a minimum value of a range of the determination window W. The end point is a maximum value of the range of the determination window W. Note that the duration and the shifting duration are information illustrated for reference, and may not necessarily be retained as the process result of step S105. This is because the shifting duration is not required in the process of the subsequent stage and the duration may be specified based on the start point and the end point.

Subsequently, the correlation determination unit 16 substitutes 1 for the variable k and substitutes the number of determination windows generated through the variable S (S106). The variable k is a variable indicating the determination window number of the determination window W to be processed. Subsequently, the correlation determination unit 16 performs a determination process on the presence or absence of a correlation between the focused event and the candidate event with respect to the determination window W having the determination window number k (hereinafter referred to as "determination window Wk") (S107). The determination process is executed based on points 1 to 4 described above. There is no particular restriction on the order of the determination windows W to be processed.

Subsequently, the correlation determination unit 16 determines whether the value of the variable k is equal to or greater than S (S108). That is, the correlation determination unit 16 determines whether step S107 has been executed for all the determination windows W. When the value of the variable k is less than S (No in S108), the correlation determination unit 16 adds 1 to the variable k (S109) and repeats the step S107. When the value of the variable k is equal to or greater than S (Yes in S108), the result output unit 17 outputs the determination result in step S107 (S110).

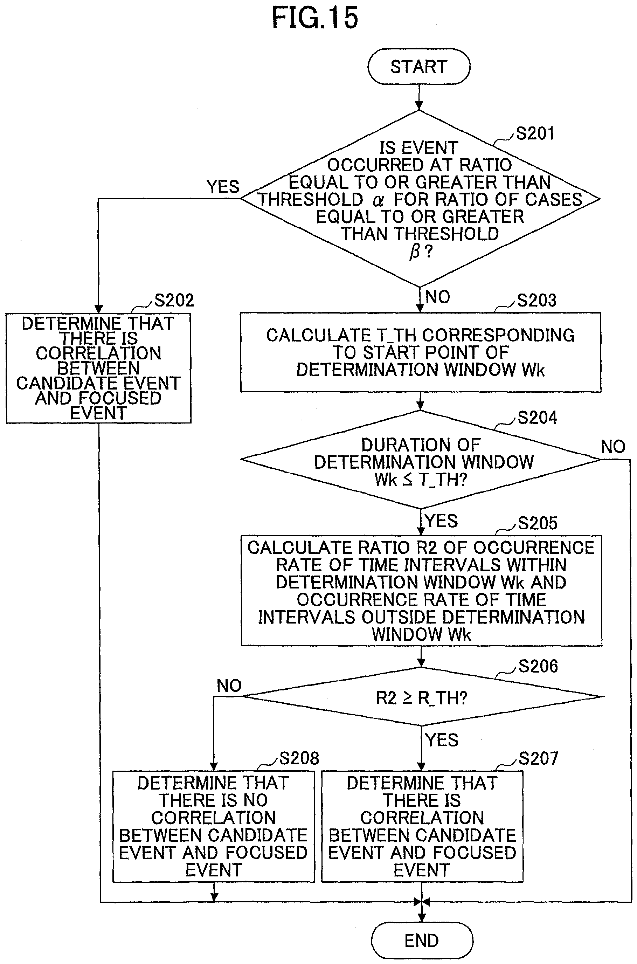

Subsequently, the details of step S107 will be described. FIG. 15 is a flowchart illustrating an example of a process of determining whether there is a correlation between a focused event and a candidate event with respect to one determination window. In the illustration of FIG. 15, the determination window Wk is the determination window Wk at the time when step S107 of FIG. 9 is executed.

In step S201, the correlation determination unit 16 executes the process related to point 2. That is, the correlation determination unit 16 calculates, for each case, the rate $Z1_{in}$ of the number of time intervals in the determination window Wk to the duration of the determination window Wk and the rate $Z1_{out}$ of the number of time intervals outside the determination window Wk to the duration outside the determination window Wk. Note that the rate $Z1_{in}$ corresponds to the occurrence rate of candidate events within the determination window Wk. Likewise, the rate $Z1_{out}$ corresponds to the occurrence rate of candidate events outside the determination window Wk. The correlation determination unit 16 determines whether a ratio K1 of cases, for which the ratio $R1 = Z1_{in}/Z1_{out}$ is equal to or greater than a threshold $\alpha$, is equal to or greater than a threshold $\beta$. More specifically, in step S201, the correlation determination unit 16 executes the following process.

First, for each case, from among the time intervals calculated in relation to the case, the correlation determination unit 16 counts the number $N(i)_{in}$ of the time intervals included in the determination window Wk, and the number $N(i)_{out}$ of the time intervals not included in the determination window Wk. Note that i is a case number. Subsequently, the correlation determination unit 16 calculates $Z1(i)_{in} = N(i)_{in}/W$ and $Z1(i)_{out} = N(i)_{out}/(W_{all} - W)$ for each case. Note that W is the duration of the determination window Wk. $W_{all}$ is the maximum time range. Subsequently, the correlation determination unit 16 calculates the ratio $R1(i) = Z1(i)_{in}/Z1(i)_{out}$ for each case. Subsequently, the correlation determination unit 16 determines whether the ratio K1 of the number of cases, where R1(i) is equal to or greater than the threshold $\alpha$, with respect to the total number of cases is greater than or equal to $\beta$.

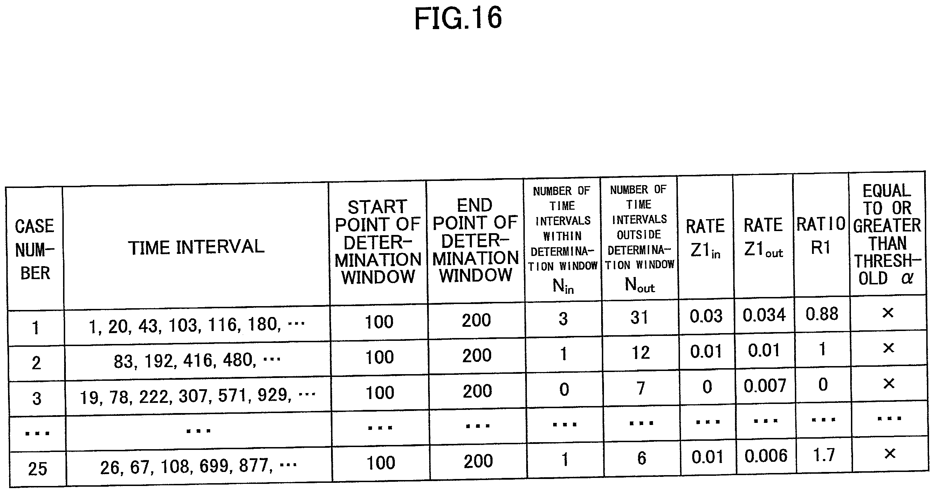

FIG. 16 is a diagram illustrating a calculation example of Z1in and $Z1_{out}$ and a ratio R1 for each case. In FIG. 16, $W_{all}$ is 1000 seconds, and the duration of the determination window Wk is 100 seconds. Further, the total number of cases is 25. Further, in FIG. 16, the threshold $\alpha$ is 8 and the threshold $\beta$ is 0.5.

For example, $N(1)_{in} = 3$, $N(1)_{out} = 31$ for a case 1 as a first example having a case number of 1. Accordingly, $Z1(1)_{in} = 3/100 = 0.03$, $Z1(1)_{out} = 31/(1000-100) = 0.034$. Further, $R1(1) = 0.03/0.034 = 0.88$. 0.88 is less than the threshold $\alpha$. Accordingly, R1(1) related to the case 1 is determined to be less than the threshold $\alpha$. Similarly, the determined results of the cases are illustrated in the rightmost columns of FIG. 16. If the number of cases determined to have the ratio R1 equal to or greater than the threshold $\alpha$ were to be 2, the ratio K1 of the number of cases (i.e., 2 cases) to the total number of cases (i.e., 25 cases) is 2/25 = 0.08. In this case, the ratio K1 is less than the threshold $\beta$. Hence, the determination in step S202 is a negative result.

When the determination result in step S202 is an affirmative result (Yes in S201), the correlation determination unit 16 determines that the candidate event has a correlation with the focused event.

However, when the determination result in step S201 is a negative result (No in S201), the correlation determination unit 16 executes a process corresponding to point 4. Specifically, the correlation determination unit 16 obtains a threshold T_TH corresponding to the start point of the determination window Wk (S203). For example, the threshold T_TH may be obtained as follows.

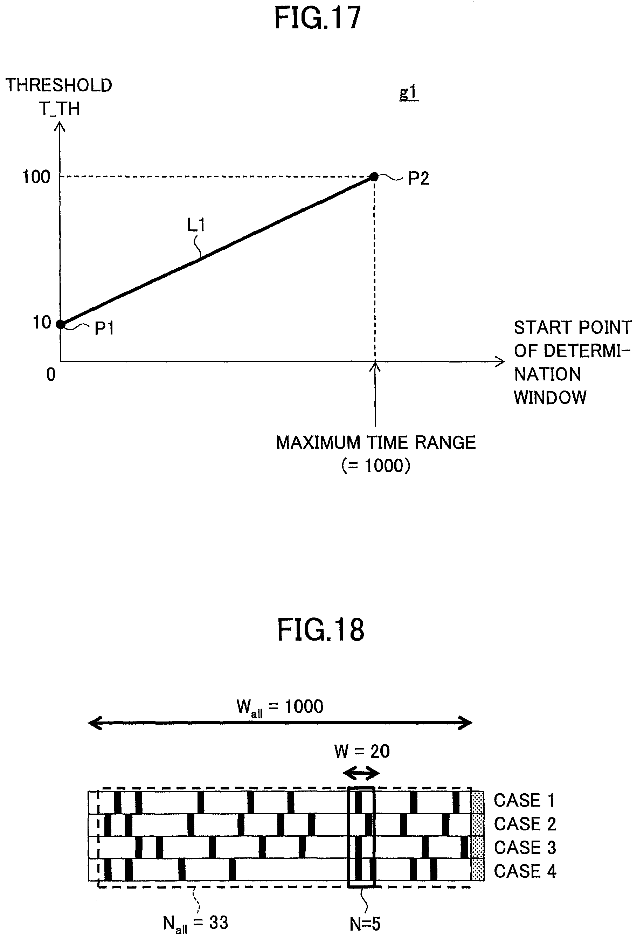

FIG. 17 is a diagram illustrating an example of a method of obtaining a threshold T_TH according to a start point of a determination window. In a graph gl in FIG. 17, the horizontal axis corresponds to the start point of the determination window W, and the vertical axis corresponds to the threshold T_TH.

First, the correlation determination unit 16 assigns the minimum value among the candidate durations of the determination window W as a threshold HT2 for the case where the start time is 0. In the present embodiment, candidate durations of the determination window W are 10, 20, 50, and 100 as illustrated in FIG. 11. Hence, the minimum value of the duration of the determination window W is 10. Thus, 10 is assigned to the case where the start time is 0. In this case, a point P1 (0,10) is arranged in the graph gl.

Subsequently, the correlation determination unit 16 assigns the maximum value among the candidate durations as the threshold T_TH, with respect to the case where the start point is the maximum time range. Accordingly, 100 is assigned to the case where the start time is 1000. In this case, a point P2 is arranged in the graph g1.

Subsequently, the correlation determination unit 16 linearly interpolates an interval between point P1 and point P2 to derive a straight line L1.

Finally, based on the straight line L1, the correlation determination unit 16 obtains a threshold T_TH corresponding to the start point of the determination window Wk.

According to the above method, the greater the value of the start time of the determination window W (i.e., the longer the occurrence time of the candidate event included in the determination window W is away from the occurrence time of the focused event), the greater the threshold T_TH may be. Note that the interpolation method between point P1 and point P2 is not limited to linear interpolation. For example, point P1 and point P2 may be connected by a curve.

Subsequently, the correlation determination unit 16 executes a process corresponding to point 3. That is, the correlation determination unit 16 determines whether the duration of the determination window Wk is equal to or less than the threshold T_TH (S204). When the duration of the determination window Wk exceeds the threshold T_TH (No in S204), the correlation determination unit 16 ends the process of FIG. 15. The determination result in this case becomes undeterminable.

However, when the duration of the determination window Wk is equal to or less than the threshold T_TH (Yes in S204), the correlation determination unit 16 executes the process corresponding to point 1. That is, among the set of the time intervals calculated in step S104 of FIG. 9, the correlation determination unit 16 calculates a ratio R2 of the occurrence rate of the time intervals included in the range of the determination window Wk to the occurrence rate of time intervals not included in the range of the determination window Wk (S205).

FIG. 18 is a diagram illustrating an example of a method of calculating the ratio R2. In FIG. 18, the maximum time range $W_{all}$ is 1000 seconds, and the duration of the determination window Wk is 20 seconds. The position (start point) of the determination window Wk is as illustrated in FIG. 18. The time interval between the candidate event and the focused event in each case is as plotted for each case in FIG. 18.

First, the correlation determination unit 16 counts the number $N_{all}$ of the time intervals calculated in step S104 of FIG. 9. In FIG. 18, $N_{all}$ = 33. In addition, the correlation determination unit 16 counts the number N of time intervals included within the range of the determination window Wk among the set of $N_{all}$ time intervals. In FIG. 18, N = 5.

Subsequently, the correlation determination unit 16 calculates a rate $Z_{in}$ of N with respect to the duration W that is the size of the range of the determination window Wk. In FIG. 18, $Z_{in}$ = 5/20 = 0.25. Subsequently, the correlation determination unit 16 calculates a rate $Z_{out}$ of the number of time intervals outside the determination window Wk to the time excluding the duration of the determination window Wk from the maximum time range $W_{all}$. In FIG. 18, $Z_{out}$ = ($N_{all}$ - N)/($W_{all}$ - W) = 0.028. Subsequently, the correlation determination unit 16 calculates a ratio R2 of $Z_{in}$ with respect to $Z_{out}$. In FIG. 18, R2 = $Z_{in}/Z_{out}$ = 8.93.

The following description is given by referring back to FIG. 15. Following step S205, the correlation determination unit 16 determines whether the ratio R2 calculated in step S205 is equal to or greater than a threshold R_TH (S206). The threshold R_TH is set in advance. For example, with reference to FIG. 18, when the threshold R_TH is 8, the ratio R2 is greater than or equal to the threshold R_TH. Accordingly, in this case (Yes in S206), the correlation determination unit 16 determines that the candidate event has a correlation with the focused event (S207). However, when the ratio R2 is less than the threshold R_TH (No in S206), the correlation determination unit 16 determines that the candidate event does not correlate with the focused event (S208).

Note that in step S110 of FIG. 9, for cases of the correlation determination unit 16 determining a correlation with any of the determination windows Wk, information indicating a focused event, information indicating a candidate event (related event) determined to have a correlation with the focused event, and the start point and the end point of the determination window Wk may be stored in the related event storage 123.

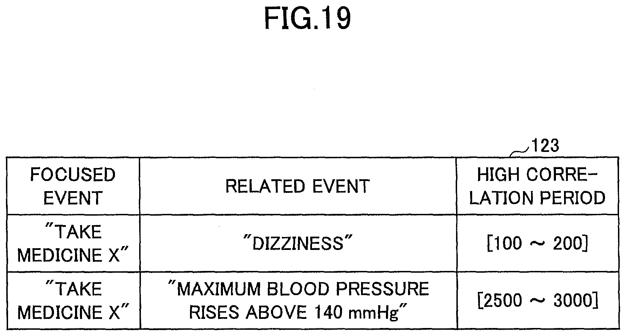

FIG. 19 is a diagram illustrating a configuration example of a related event storage. In FIG. 19, the related event storage 123 stores information indicating a focused event, information indicating a related event, and a high correlation period in association with one another. The high correlation period is information indicating the start point and the end point of the determination window Wk determined to have a high correlation with respect to the focused event and the related event.

When the correlation determination unit 16 determines that there is a correlation with respect to two or more determination windows Wk, the start point and the end point of the determination window Wk having the highest ratio R2 may be output. Alternatively, based on the ratio R, the start point and the end point of each determination window Wk may be sorted and output. When the correlation determination unit 16 determines that there is no correlation with respect to all the determination windows Wk, information indicating that there is no correlation between the focused event and the candidate event may be output.

In the above description, when the correlation determination unit 16 determines that there is a correlation with respect to at least one determination window Wk, there is a correlation between the focused event and the candidate event. However, as a requirement for recognizing the presence of a correlation between the focused event and the candidate event, there may be an additional requirement that the number of determination windows Wk determined to have the correlation does not exceed a predetermined threshold $\gamma$. In this case, information (start point and end point, etc.) on all the determination windows Wk determined to have correlation may be output. However, when there are two or more determination windows Wk having overlapping ranges, for example, only information on the determination window Wk in which R2 or K1 is the maximum may be output.

When the number of the determination windows Wk determined to have the correlation exceeds the threshold γ, values such as R_TH, α, β, and the like, which are thresholds for determining the presence or absence of a correlation, may be reviewed. For example, each threshold may be set by a method as described below. After the review of the threshold, steps subsequent to step S106 in FIG. 9 may be executed.

An example of a method of determining the threshold R_TH and the threshold α will be described. With respect to the illustration of FIG. 18, the following formula (1)is established with respect to the relationship between $Z_{in}/Z_{out}$ and the threshold R_TH.

$$\frac{N}{W} > R\_TH\left(\frac{N_{all}-N}{W_{all}-W}\right) \cdots (1)$$

In the formula (1), the left side corresponds to $Z_{in}$. The inside of the parentheses on the right side corresponds to $Z_{out}$.

With respect to the illustration of FIG. 16, the following formula (2) is established for the relationship between $Z1(i)_{in}/Z1(i)_{out}$ and the threshold α.

$$\frac{N(i)_{in}}{W} > \alpha\left(\frac{N(i)_{out}}{W_{all}-W}\right) = \alpha\left(\frac{N_{all}-N(i)_{in}}{W_{all}-W}\right) \cdots (2)$$

Basically, from the relationship between the formula (1) and the formula (2), the same value set for the threshold R_TH may be set for the threshold α. Then, the threshold R_TH may be determined based on the ratio R2 calculated using the known data, for example, on two event types that are known to have clear correlation.

For example, it is assumed that it is known that the focused event X and the related event Y have a correlation. Further, it is assumed that the relevant time range is also known, and it is known that the probability of occurrence of the related event Y 10 to 20 minutes before the focused event occurs is high.

Under such circumstances, as described above, using the known event data of the focused event X and the related event Y, a time interval is calculated, for each case, between the focused event X related to the case and each of the related events Y within the maximum time range from the focused event X.

Subsequently, the occurrence rate $Z_{in}$ of the related event in the determination window W and the occurrence rate $Z_{out}$ of the related event outside the determination window W are obtained using the determination window W related to the relevant time range (10 to 20 minutes beforehand), and then the ratio R2 = $Z_{in}/Z_{out}$ is obtained.

Since the value of R_TH is the lowest threshold, the value of R_TH may be set to have at least the value of the ratio R2 or more. Note that in the determination of the threshold value R_TH, R2(p) may be calculated for each person p using known data of two or more persons, and the maximum value of R2(p) may be set as R2.

As described above, according to the present embodiment, for each of the determination windows W, the occurrence rate of the candidate event in the determination window W is compared with the occurrence rate of the candidate events outside the determination window W, and the presence or absence of a correlation between the focused event and the candidate event is determined. Accordingly, the accuracy in extracting events having a correlation with a certain event will be much improved, compared to the case where the presence or absence of a correlation between the focused event and the candidate event is simply determined based on the frequency or the like.

Note that this embodiment may be applied to events (events) other than events related to daily life of a person. For example, the embodiment may be applied to an event indicating a state of an apparatus. In addition, the embodiment may be applied to an event (log information) output from a computer system.

In the present embodiment, the focused event is an example of a first type event. The candidate event is an example of a second type event. The time interval calculator 14 is an example of a calculator. The correlation determination unit 16 is an example of a determination unit.

The embodiments of the present invention have been described in detail above; however, the present invention is not limited to a specific one of the embodiments, and various modifications and changes may be made within the scope described in the claims. Although the embodiments of the present invention have been described in detail above, the present invention is not limited to such specific embodiments, and various modifications and changes may be made within the scope of the gist of the invention described in the claims. The threshold value α is an example of the first threshold value. The threshold value β is an example of the second threshold value. The threshold value T_TH is an example of the third threshold value.

The present application claims its priority based on Japanese Patent Application No. 2015-035389 filed on February 25, 2015, and the entire contents of the same Japanese patent application are incorporated by reference in the present application.

EXPLANATION OF LETTERS OR NUMERALS

| | |
|---|---|
| 10 | correlation determination apparatus |
| 11 | event selector |
| 12 | event data acquisition unit |
| 13 | parameter acquisition unit |
| 14 | time interval calculator |
| 15 | determination window generator |
| 16 | correlation determination unit |
| 17 | result output unit |
| 100 | drive device |
| 101 | recoding medium |
| 102 | auxiliary storage device |
| 103 | memory device |
| 104 | CPU |
| 105 | interface device |
| 121 | event data storage |
| 122 | parameter storage |
| 123 | related event storage |
| B | bus |

**Claims**

1. A correlation determination program for causing a computer to execute a process comprising:

   calculating a time interval between an occurrence time of one of first type events and an occurrence time of each of second type events based on information including the occurrence times of the first type events and the second type events, on a first type event basis, each of the second type events occurring within a predetermined time from a corresponding one of the occurrence times of the first type events;
   comparing, for each of a plurality of ranges of values relating to the time interval, the plurality of ranges differing from one another in at least one of a minimum value and a maximum value, a first rate of the number of time intervals within a corresponding one of ranges among a set of time intervals with respect to a size of the range and a second rate of the number of time intervals outside the corresponding range among the set of time intervals with respect to a size of a range obtained by excluding the range from the predetermined time; and
   determining presence or absence of a correlation between the first type event and the second type event.

2. The program as claimed in claim 1, wherein
   the determining includes
   calculating a ratio of a third rate to a fourth rate for each of the first type events with respect to each of the plurality of ranges, the third rate being a rate of the number of time intervals within the range, among the set of time intervals calculated for the first type event, with respect to the size of the range, the fourth rate being a rate of the number of time intervals outside the range among the set of time intervals calculated for the first type event, with respect to a size of a range obtained by excluding the range from the predetermined time; and
   determining that there is a correlation between the first type event and the second type event irrespective of the comparison between the first rate and the second rate, in response to the number of ratios equal to or greater than a first threshold being equal to or greater than a second threshold with respect to any one of the plurality of ranges, among the set of the ratios calculated for the range.

3. The program as claimed in claim 1 or 2, wherein
   the determining includes comparing the first rate and the second rate with respect to a range having length being equal to or less than a third threshold among the plurality of ranges.

4. The program as claimed in claim 3, wherein
   the determining includes relatively increasing the third threshold with respect to a range having a relatively large minimum value.

5. A correlation determination method executed by a computer, the correlation determination method comprising:

calculating a time interval between an occurrence time of one of first type events and an occurrence time of each of second type events based on information including the occurrence times of the first type events and the second type events, on a first type event basis, each of the second type events occurring within a predetermined time from a corresponding one of the occurrence times of the first type events;

comparing, for each of a plurality of ranges of values relating to the time interval, the plurality of ranges differing from one another in at least one of a minimum value and a maximum value, a first rate of the number of time intervals within a corresponding one of ranges among a set of time intervals with respect to a size of the range and a second rate of the number of time intervals outside the corresponding range among the set of time intervals with respect to a size of a range obtained by excluding the range from the predetermined time; and

determining presence or absence of a correlation between the first type event and the second type event.

6. The correlation determination method as claimed in claim 5, wherein
the determining includes

calculating a ratio of a third rate to a fourth rate for each of the first type events with respect to each of the plurality of ranges, the third rate being a rate of the number of time intervals within the range, among the set of time intervals calculated for the first type event, with respect to the size of the range, the fourth rate being a rate of the number of time intervals outside the range among the set of time intervals calculated for the first type event, with respect to a size of a range obtained by excluding the range from the predetermined time; and

determining that there is a correlation between the first type event and the second type event irrespective of the comparison between the first rate and the second rate, in response to the number of ratios equal to or greater than a first threshold being equal to or greater than a second threshold with respect to any one of the plurality of ranges, among the set of the ratios calculated for the range.

7. The correlation determination method as claimed in claim 5 or 6, wherein
the determining includes comparing the first rate and the second rate with respect to a range having length being equal to or less than a third threshold among the plurality of ranges.

8. The correlation determination method as claimed in claim 7, wherein
the determining includes relatively increasing the third threshold with respect to a range having a relatively large minimum value.

9. A correlation determination apparatus comprising:

a calculator configured to calculate a time interval between an occurrence time of one of first type events and an occurrence time of each of second type events based on information including the occurrence times of the first type events and the second type events, on a first type event basis, each of the second type events occurring within a predetermined time from a corresponding one of the occurrence times of the first type events; and

a determination unit configured to compare, for each of a plurality of ranges of values relating to the time interval, the plurality of ranges differing from one another in at least one of a minimum value and a maximum value, a first rate of the number of time intervals within a corresponding one of ranges among a set of time intervals with respect to a size of the range and a second rate of the number of time intervals outside the corresponding range among the set of time intervals with respect to a size of a range obtained by excluding the range from the predetermined time to determine presence or absence of a correlation between the first type event and the second type event.

10. The correlation determination apparatus as claimed in claim 9, wherein
the determination unit

calculates a ratio of a third rate to a fourth rate for each of the first type events with respect to each of the plurality of ranges, the third rate being a rate of the number of time intervals within the range, among the set of time intervals calculated for the first type event, with respect to the size of the range, the fourth rate being a rate of the number of time intervals outside the range among the set of time intervals calculated for the first type event, with respect to a size of a range obtained by excluding the range from the predetermined time; and

determines that there is a correlation between the first type event and the second type event irrespective of the comparison between the first rate and the second rate, in response to the number of ratios equal to or greater than a first threshold being equal to or greater than a second threshold with respect to any one of the plurality of ranges, among the set of the ratios calculated for the range.

11. The correlation determination apparatus as claimed in claim 9 or 10, wherein
    the determination unit compares the first rate and the second rate with respect to a range having length being equal to or less than a third threshold among the plurality of ranges.

12. The correlation determination apparatus as claimed in claim 11, wherein
    the determination unit relatively increases the third threshold with respect to a range having a relatively large minimum value.

# FIG.1

CANDIDATE EVENT

FOCUSED EVENT

09:00:00
JULY 28,
2014

12:00:00
JULY 28,
2014

CASE 1

15:00:00
JULY 28,
2014

CASE 2

18:00:00
JULY 28,
2014

CASE 3

(1)

90 MIN.
AGO

60 MIN.
AGO

30 MIN.
AGO

0 MIN.
AGO

CASE 1

CASE 2

CASE 3

CASE 4

CASE 5

CASE 6

CASE 7

CASE 8

W

(2)

EP 3 264 347 A1

# FIG.2

# FIG.3

# FIG.4

EP 3 264 347 A1

| 300 MIN. AGO | ... | 90 MIN. AGO | 60 MIN. AGO | 30 MIN. AGO | 0 MIN. AGO | 300 MIN. AGO | ... | 90 MIN. AGO | 60 MIN. AGO | 30 MIN. AGO | 0 MIN. AGO |

W

INTERVAL
BETWEEN EVENTS

W

INTERVAL
BETWEEN EVENTS

# FIG.5

# FIG.6

# FIG.7

CPU ~104

INTERFACE DEVICE ~105

10

B

DRIVE DEVICE ~100

AUXILIARY STORAGE DEVICE ~102

MEMORY DEVICE ~103

RECORDING MEDIUM ~101

# FIG.8

CORRELATION DETERMINATION APPARATUS 10

| 11 EVENT SELECTOR | 12 EVENT DATA ACQUISITION UNIT | 13 PARAMETER ACQUISI-TION UNIT | 14 TIME INTERVAL CALCULATOR | 15 DETERMINA-TION WINDOW GENERATOR | 16 CORRELA-TION DETERMINA-TION UNIT | 17 RESULT OUTPUT UNIT |

121 EVENT DATA STORAGE

122 PARAMETER STORAGE

123 RELATED EVENT STORAGE

EP 3 264 347 A1

# FIG.9

START

S101

SELECT FOCUSED EVENT AND RELATED EVENT

S102

ACQUIRES TIME SERIES DATA OF TWO EVENT TYPES

S103

ACQUIRE PARAMETER RELATED TO DETERMINATION WINDOW

S104

CALCULATE TIME INTERVAL BETWEEN FOCUSED EVENT AND EACH OF CANDIDATE EVENTS, FOR EACH FOCUSED EVENT

S105

GENERATE DETERMINATION WINDOW

S106

$k \leftarrow 1$; $S \leftarrow$ NUMBER OF DETERMINATION WINDOWS

S107

DETERMINE PRESENCE OR ABSENCE OF CORRELATION BETWEEN FOCUSED EVENT AND CANDIDATE EVENT IN RELATION TO DETERMINATION WINDOW Wk

S108

$k \geq S?$

NO

S109

k++

YES

S110

OUTPUT DETERMINATION RESULT

END

22

# FIG.10

| CASE NUMBER | OCCURRENCE DATE AND TIME OF FOCUSED EVENT | OCCUR-RENCE NUMBER | OCCURRENCE DATE AND TIME OF CANDIDATE EVENT |
|---|---|---|---|
| 1 | 10:00:00 DECEMBER 18, 2014 | 1 | 10:00:01 DECEMBER 18, 2014 |
| 2 | 13:10:03 DECEMBER 18, 2014 | 2 | 10:00:20 DECEMBER 18, 2014 |
| 3 | 20:02:19 DECEMBER 18, 2014 | 3 | 10:00:43 DECEMBER 18, 2014 |
| 4 | 08:34:54 DECEMBER 19, 2014 | 4 | 10:01:43 DECEMBER 18, 2014 |
| ... | ... | ... | ... |

(1)                                                    (2)

# FIG.11

⌐122

| DURATION CANDIDATE | MAGNIFICATION OF SHIFTING DURATION | MAXIMUM TIME RANGE |
|---|---|---|
| 10, 20, 50, 100 | 0.5 | 1000 |

# FIG.12

| CASE NUMBER | OCCURRENCE DATE AND TIME OF FOCUSED EVENT | TIME INTERVAL BETWEEN OCCURRENCE OF FOCUSED EVENT AND OCCURRENCE OF CANDIDATE EVENT |
|---|---|---|
| 1 | 10:00:00 DECEMBER 18, 2014 | 1, 20, 43, 103, 116, 180, ··· |
| 2 | 13:10:03 DECEMBER 18, 2014 | 83, 192, 416, 480, ··· |
| 3 | 20:02:19 DECEMBER 18, 2014 | 19, 78, 222, 307, 571, 929, ··· |
| 4 | 08:34:54 DECEMBER 19, 2014 | 107, 250, 259, 671, 730, 799, ··· |
| ··· | ··· | ··· |

# FIG.13

DURATION ← 0

10   □   ...   □ □ □   ...   □ □ □ □ □

20   ▭   ...   ▭   ...   ▭ ▭ ▭

50   ▭   ...   ▭   ▭

100   ▭   ...   ▭

## FIG.14

| DETERMINATION WINDOW NUMBER | DURATION | SHIFTING DURATION | START POINT | END POINT | |
|---|---|---|---|---|---|
| 1 | 10 | 5 | 0 | 10 | ⎫ |
| 2 | 10 | 5 | 5 | 15 | ⎬ ALL DETERMINATION WINDOWS WHEN DURATION = 10 |
| ... | ... | ... | ... | ... | |
| 200 | 10 | 5 | 990 | 1000 | ⎭ |
| 201 | 20 | 10 | 0 | 20 | ⎫ |
| 202 | 20 | 10 | 10 | 30 | ⎬ ALL DETERMINATION WINDOWS WHEN DURATION = 20 |
| ... | ... | ... | ... | ... | |
| 300 | 20 | 10 | 980 | 1000 | ⎭ |
| 301 | 50 | 25 | 0 | 50 | |
| ... | ... | ... | ... | ... | |
| N | 100 | 50 | 950 | 1000 | |

EP 3 264 347 A1

# FIG.15

START

S201
IS EVENT OCCURRED AT RATIO EQUAL TO OR GREATER THAN THRESHOLD $\alpha$ FOR RATIO OF CASES EQUAL TO OR GREATER THAN THRESHOLD $\beta$ ?

YES

S202
DETERMINE THAT THERE IS CORRELATION BETWEEN CANDIDATE EVENT AND FOCUSED EVENT

NO

S203
CALCULATE T_TH CORRESPONDING TO START POINT OF DETERMINATION WINDOW Wk

S204
DURATION OF DETERMINATION WINDOW Wk ≤ T_TH?

NO

YES

S205
CALCULATE RATIO R2 OF OCCURRENCE RATE OF TIME INTERVALS WITHIN DETERMINATION WINDOW Wk AND OCCURRENCE RATE OF TIME INTERVALS OUTSIDE DETERMINATION WINDOW Wk

S206
R2 ≥ R_TH?

NO

YES

S208
DETERMINE THAT THERE IS NO CORRELATION BETWEEN CANDIDATE EVENT AND FOCUSED EVENT

S207
DETERMINE THAT THERE IS CORRELATION BETWEEN CANDIDATE EVENT AND FOCUSED EVENT

END

# FIG.16

| CASE NUM-BER | TIME INTERVAL | START POINT OF DETER-MINATION WINDOW | END POINT OF DETER-MINATION WINDOW | NUMBER OF TIME INTERVALS WITHIN DETERMINA-TION WINDOW $N_{in}$ | NUMBER OF TIME INTERVALS OUTSIDE DETERMINA-TION WINDOW $N_{out}$ | RATE $Z1_{in}$ | RATE $Z1_{out}$ | RATIO R1 | EQUAL TO OR GREATER THAN THRESH-OLD $\alpha$ |
|---|---|---|---|---|---|---|---|---|---|
| 1 | 1, 20, 43, 103, 116, 180, ⋯ | 100 | 200 | 3 | 31 | 0.03 | 0.034 | 0.88 | × |
| 2 | 83, 192, 416, 480, ⋯ | 100 | 200 | 1 | 12 | 0.01 | 0.01 | 1 | × |
| 3 | 19, 78, 222, 307, 571, 929, ⋯ | 100 | 200 | 0 | 7 | 0 | 0.007 | 0 | × |
| ⋯ | ⋯ | ⋯ | ⋯ | ⋯ | ⋯ | ⋯ | ⋯ | ⋯ | ⋯ |
| 25 | 26, 67, 108, 699, 877, ⋯ | 100 | 200 | 1 | 6 | 0.01 | 0.006 | 1.7 | × |

EP 3 264 347 A1

# FIG.17

THRESHOLD
T_TH

$\underline{g1}$

100 ············· P2

L1

10 P1

0

START POINT
OF DETERMI-
NATION
WINDOW

MAXIMUM TIME RANGE
(= 1000)

# FIG.18

$W_{all} = 1000$

$W = 20$

CASE 1
CASE 2
CASE 3
CASE 4

$N_{all} = 33$        $N=5$

# FIG.19

123

| FOCUSED EVENT | RELATED EVENT | HIGH CORRE-LATION PERIOD |
|---|---|---|
| "TAKE MEDICINE X" | "DIZZINESS" | [100 ~ 200] |
| "TAKE MEDICINE X" | "MAXIMUM BLOOD PRESSURE RISES ABOVE 140 mmHg" | [2500 ~ 3000] |

## INTERNATIONAL SEARCH REPORT

| International application No. |
|---|
| PCT/JP2015/082915 |

A. CLASSIFICATION OF SUBJECT MATTER
*G06Q50/22*(2012.01)i, *G06Q50/24*(2012.01)i, *A61B5/00*(2006.01)i

According to International Patent Classification (IPC) or to both national classification and IPC

B. FIELDS SEARCHED

Minimum documentation searched (classification system followed by classification symbols)
G06Q10/00-50/34, A61B5/00

Documentation searched other than minimum documentation to the extent that such documents are included in the fields searched

| | | | |
|---|---|---|---|
| Jitsuyo Shinan Koho | 1922-1996 | Jitsuyo Shinan Toroku Koho | 1996-2016 |
| Kokai Jitsuyo Shinan Koho | 1971-2016 | Toroku Jitsuyo Shinan Koho | 1994-2016 |

Electronic data base consulted during the international search (name of data base and, where practicable, search terms used)

C. DOCUMENTS CONSIDERED TO BE RELEVANT

| Category* | Citation of document, with indication, where appropriate, of the relevant passages | Relevant to claim No. |
|---|---|---|
| A | JP 2007-175225 A (Sumitomo Osaka Cement Co., Ltd., Keio University), 12 July 2007 (12.07.2007), entire text; all drawings (Family: none) | 1-12 |
| A | JP 2011-508330 A (Koninklijke Philips Electronics N.V.), 10 March 2011 (10.03.2011), entire text; all drawings & US 2010/0324938 A1 entire text; all drawings & WO 2009/083839 A1 & EP 2240877 A1 & CN 101911079 A | 1-12 |

☒ Further documents are listed in the continuation of Box C.  ☐ See patent family annex.

| | |
|---|---|
| * Special categories of cited documents: | "T" later document published after the international filing date or priority date and not in conflict with the application but cited to understand the principle or theory underlying the invention |
| "A" document defining the general state of the art which is not considered to be of particular relevance | |
| "E" earlier application or patent but published on or after the international filing date | "X" document of particular relevance; the claimed invention cannot be considered novel or cannot be considered to involve an inventive step when the document is taken alone |
| "L" document which may throw doubts on priority claim(s) or which is cited to establish the publication date of another citation or other special reason (as specified) | "Y" document of particular relevance; the claimed invention cannot be considered to involve an inventive step when the document is combined with one or more other such documents, such combination being obvious to a person skilled in the art |
| "O" document referring to an oral disclosure, use, exhibition or other means | |
| "P" document published prior to the international filing date but later than the priority date claimed | "&" document member of the same patent family |

| Date of the actual completion of the international search | Date of mailing of the international search report |
|---|---|
| 19 February 2016 (19.02.16) | 01 March 2016 (01.03.16) |

| Name and mailing address of the ISA/ | Authorized officer |
|---|---|
| Japan Patent Office 3-4-3,Kasumigaseki,Chiyoda-ku, Tokyo 100-8915,Japan | |
| | Telephone No. |

Form PCT/ISA/210 (second sheet) (January 2015)

**INTERNATIONAL SEARCH REPORT**

International application No.

PCT/JP2015/082915

C (Continuation).   DOCUMENTS CONSIDERED TO BE RELEVANT

| Category* | Citation of document, with indication, where appropriate, of the relevant passages | Relevant to claim No. |
|---|---|---|
| A | JP 2012-532633 A  (Cameron Health, Inc.),<br>20 December 2012 (20.12.2012),<br>entire text; all drawings<br>& US 2010/0331904 A1<br>entire text; all drawings<br>& WO 2011/008550 A1     & EP 2459275 A1<br>& CN 102596310 A | 1-12 |
| A | JP 2011-519289 A  (Deepbreeze Ltd.),<br>07 July 2011 (07.07.2011),<br>entire text; all drawings<br>& US 2011/0034818 A1<br>entire text; all drawings<br>& WO 2009/125407 A1     & EP 2262416 A1<br>& CN 102149317 A | 1-12 |
| A | JP 2007-48200 A  (The Chugoku Electric Power<br>Co., Inc.),<br>22 February 2007 (22.02.2007),<br>entire text; all drawings<br>(Family: none) | 1-12 |
| A | WO 2007/143234 A2  (MEDTRONIC, INC.),<br>13 December 2007 (13.12.2007),<br>entire text; all drawings<br>& US 2008/0269835 A1     & EP 2012659 A2 | 1-12 |

Form PCT/ISA/210 (continuation of second sheet) (January 2015)

**REFERENCES CITED IN THE DESCRIPTION**

**Patent documents cited in the description**

- JP 2008041041 A
- JP 2004157614 A
- JP 2007048200 A
- JP 2013131170 A
- JP 2001540710 A
- JP 2011209908 A
- JP 2015035389 A